(19) Europäisches Patentamt
European Patent Office
Office européen des brevets

(11) **EP 4 324 867 A1**

(12) **EUROPEAN PATENT APPLICATION**
published in accordance with Art. 153(4) EPC

(43) Date of publication:
**21.02.2024 Bulletin 2024/08**

(21) Application number: **22804481.4**

(22) Date of filing: **14.04.2022**

(51) International Patent Classification (IPC):
**C08J 3/12** *(2006.01)*

(52) Cooperative Patent Classification (CPC):
**C08J 3/12**

(86) International application number:
**PCT/JP2022/017873**

(87) International publication number:
**WO 2022/244567 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.05.2021 JP 2021083790**

(71) Applicant: **Sumitomo Seika Chemicals Co., Ltd.
Kako-gun, Hyogo 675-0145 (JP)**

(72) Inventors:
- **YAMAMOTO Tomoka
  Himeji-shi, Hyogo 672-8076 (JP)**
- **SAWAKI Hiroki
  Himeji-shi, Hyogo 672-8076 (JP)**

(74) Representative: **Vossius & Partner
Patentanwälte Rechtsanwälte mbB
Siebertstrasse 3
81675 München (DE)**

(54) **WATER-ABSORBING RESIN PARTICLE, ABSORBER, AND ABSORBENT ARTICLE**

(57) The present invention relates to a water-absorbent resin particle, in which when a lock-up height at an n-minute value is denoted by Hn [cm], the water-absorbent resin particle has H1 of 1.0 cm or less and H10/H1 of 5.0 or more.

EP 4 324 867 A1

**Description**

**Technical Field**

The present invention relates to a water-absorbent resin particle, an absorber, and an absorbent article.

**Background Art**

Water-absorbent resin particles are widely used in various fields of sanitary materials such as disposable diapers, hygiene products, and portable toilets; agricultural and horticultural materials such as water retention agents and soil improvement agents; and industrial materials such as waterproofing agents and condensation prevention agents. It is desirable that an absorber containing the water-absorbent resin particles has a better absorption rate of humor and the like to be absorbed. Regarding the absorber containing the water-absorbent resin particles, for example, it is disclosed in Patent Literature 1 that an absorber containing water-absorbing resin powder includes a layer, at least a part of the layer having a diffusible region where a material for improving diffusibility with a liquid permeation speed under load of 15 seconds or less is disposed.

**Citation List**

**Patent Literature**

[Patent Literature 1] Japanese Unexamined Patent Publication No. 2014-46020

**Summary of Invention**

**Technical Problem**

Usability of an absorber included in a sanitary product (absorbent article) such as a diaper varies depending on a state at the time of wearing the absorbent article (roughly classified into a laying state such as a bed time and a standing state when active). According to diligent research conducted by the present inventors, it has been found that such a variation in the usability of the absorber is because a main part in which the absorber is used varies depending on a state at the time of wearing. That is, the absorber is used such that a plane (flat) portion thereof is mainly used in a laid state, and a curved portion in a U-shape is mainly used in a standing state. In the plane portion of the absorber, as the number of urination times increases, a gel blocking phenomenon may occur, and an absorption rate may decrease. Therefore, in a case where the plane portion of the absorber is mainly used, the water-absorbent resin particle is required to have liquid permeability. On the other hand, in a case where the curved portion of the absorber is mainly used, the water-absorbent resin particle is required to have a sufficient water absorption capacity because a liquid is necessary to be absorbed against gravity. However, in general, it is difficult to produce water-absorbent resin particles having a high permeation rate in both the plane portion and the curved portion by achieving both of the liquid permeability and the water absorption capacity because the liquid permeability and the water absorption capacity are in a trade-off relationship.

An object of the present invention is to provide novel water-absorbent resin particles for obtaining an absorbent article that enables absorption of moisture at a sufficiently high permeation rate in both the plane portion and the curved portion with conventional water-absorbent resin particles used in combination.

**Solution to Problem**

One aspect of the present invention is to provide a water-absorbent resin particle, in which when a lock-up height at an n-minute value is denoted by Hn [cm], H1 is 1.0 cm or less, and H10/H1 is 5.0 or more. The lock-up height at the n-minute value is measured by the following procedure.

(1) 0.200 g of water-absorbent resin particles is disposed over an entire bottom surface in a cylinder having an inner diameter of 2.0 cm and a depth of 8.0 cm to form a particle layer, and a height of the particle layer is denoted by H0 [cm].
(2) 20 g of physiological saline is injected to the particle layer to swell the particle layer.
(3) A height of the particle layer when n minutes have passed from the injection of the total amount of the physiological saline is recorded as Hn', and a lock-up height Hn [cm] at an n-minute value is calculated from an expression represented by Expression: Hn' - H0.

Another aspect of the present invention is to provide a water-absorbent resin composition containing the above-mentioned water-absorbent resin particle and a water-absorbent resin particle other than the water-absorbent resin particle.

Still another aspect of the present invention is to provide an absorber containing the above-mentioned water-absorbent resin particle or the water-absorbent resin composition.

Even still another aspect of the present invention is to provide an absorbent article including the above-mentioned absorber.

When H1 of the water-absorbent resin particle is low, it is shown that rapid water absorption is suppressed in the initial stage of water absorption. When H10/H1 of the water-absorbent resin particle is high, it is shown that rapid water absorption is suppressed in the initial stage of water absorption, but high water absorption power is exhibited at a stage after a lapse of a predetermined time from the initiation of water absorption. The present inventors have found that adjustment of H1 and H10/H1 within the above-mentioned range improves both a permeation rate of a liquid in the plane portion of the absorber and a permeation rate of a liquid in the curved portion of the absorber, thereby reaching the completion of the present invention.

**Advantageous Effects of Invention**

According to the present invention, it is possible to provide novel water-absorbent resin particles for obtaining an absorbent article that enables absorption of moisture at a sufficiently high permeation rate in both the plane portion and the curved portion with conventional water-absorbent resin particles used in combination.

**Brief Description of Drawings**

Fig. 1 is a cross-sectional view showing an embodiment of an absorbent article.
Fig. 2 is a schematic diagram showing a method for measuring non-pressurization DW.
Fig. 3 is a schematic diagram showing a water absorption test method for evaluating a permeation rate of a liquid in a curved portion of an absorber.
Fig. 4 is scanning electron microscope (SEM) photographs of water-absorbent resin particles of Example 1 and Example 2.
Fig. 5 is SEM photographs of water-absorbent resin particles of Example 3, Example 4, and Example 5.
Fig. 6 is SEM photographs of water-absorbent resin particles of Comparative Example 2 and Comparative Example 3.

**Description of Embodiments**

Hereinafter, some embodiments of the present invention will be described in detail. However, the present invention is not limited to the following embodiments.

In the present specification, "(meth)acryl" means both acryl and methacryl. "Acrylate" and "methacrylate" are also referred to as "(meth)acrylate". The same also applies to other similar terms. In a numerical value range described in a stepwise manner in the present specification, an upper limit value or a lower limit value of a numerical value range in a certain step can be optionally combined with an upper limit value or a lower limit value of a numerical value range in another step. In a numerical value range described in the present specification, the upper limit value or the lower limit value of the numerical value range may be replaced with the value shown in the examples. The materials exemplified in the present specification may be used alone or in combination of two or more.

When a lock-up height at an n-minute value is denoted by Hn [cm], a water-absorbent resin particle according to the present embodiment has H1 of 1.0 cm or less and H10/H1 of 5.0 or more.

The lock-up height at the n-minute value is measured by the following procedure.

(1) 0.200 g of water-absorbent resin particles is disposed over an entire bottom surface in a cylinder having an inner diameter of 2.0 cm and a depth of 8.0 cm to form a particle layer, and a height of the particle layer is denoted by H0 [cm].
(2) 20 g of physiological saline is injected to the particle layer to swell the particle layer.
(3) A height of the particle layer when n minutes have passed from the injection of a total amount of the physiological saline is recorded as Hn', and a lock-up height Hn [cm] at an n-minute value is calculated from an expression represented by Hn' - H0. The physiological saline is an aqueous solution containing saline having a concentration of 0.9% by mass, based on a volume (mL) of the physiological saline. Other details of the test conditions will be described in Examples described later.

H1 is a lock-up height at a 1-minute value. From the viewpoint of further improving a permeation rate of a liquid in the plane portion of the absorber and the viewpoint of further improving a permeation rate of a liquid into the absorber, H1 may be 0.9 cm or less, 0.8 cm or less, 0.7 cm or less, 0.6 cm or less, 0.5 cm or less, or 0.4 cm or less. The lower limit of the H1 is not particularly limited, but may be 0.1 cm or more, 0.2 cm or more, or 0.3 cm or more. H1 may be 0.1 cm or more and 1.0 cm or less, and from the viewpoint of further improving a permeation rate of a liquid in the plane portion of the absorber and the viewpoint of further improving a permeation rate of a liquid into the absorber, H1 may be 0.1 cm or more and 0.9 cm or less, 0.1 cm or more and 0.8 cm or less, 0.1 cm or more and 0.7 cm or less, 0.1 cm or more and 0.6 cm or less, 0.1 cm or more and 0.5 cm or less, or 0.1 cm or more and 0.4 cm or less.

H5 is a lock-up height at a 5-minute value. H5 may be 1.0 cm or more, 1.5 cm or more, 2.0 cm or more, 2.5 cm or more, 3.0 cm or more, 3.5 cm or more, 4.0 cm or more, or 4.5 cm or more. H5 may be 10.0 cm or less, 9.5 cm or less, 9.0 cm or less, 8.5 cm or less, 8.0 cm or less, 7.5 cm or less, 7.0 cm or less, 6.5 cm or less, 6.0 cm or less, 5.5 cm or less, or 5.0 cm or less. H5 may be 1.0 cm or more and 10.0 cm or less, 1.0 cm or more and 9.5 cm or less, 1.5 cm or more and 9.0 cm or less, 1.5 cm or more and 8.5 cm or less, 2.0 cm or more and 8.0 cm or less, 2.0 cm or more and 7.5 cm or less, 2.5 cm or more and 7.0 cm or less, 3.0 cm or more and 6.5 cm or less, 3.5 cm or more and 6.0 cm or less, 4.0 cm or more and 5.5 cm or less, or 4.5 cm or more and 5.0 cm or less.

H10 is a lock-up height at a 10-minute value. H10 may be 1.0 cm or more, 1.5 cm or more, 2.0 cm or more, 2.5 cm or more, 3.0 cm or more, 3.5 cm or more, 4.0 cm or more, 4.5 cm or more, or 5.0 cm or more. H10 may be 10.0 cm or less, 9.5 cm or less, 9.0 cm or less, 8.5 cm or less, 8.0 cm or less, 7.5 cm or less, 7.0 cm or less, 6.5 cm or less, 6.0 cm or less, or 5.5 cm or less. H10 may be 1.0 cm or more and 10.0 cm or less, 1.0 cm or more and 9.5 cm or less, 1.5 cm or more and 9.0 cm or less, 2.0 cm or more and 8.5 cm or less, 2.5 cm or more and 8.0 cm or less, 3.0 cm or more and 7.5 cm or less, 3.5 cm or more and 7.0 cm or less, 4.0 cm or more and 6.5 cm or less, 4.5 cm or more and 6.0 cm or less, or 5.0 cm or more and 5.5 cm or less.

From the viewpoint of further improving a permeation rate of a liquid in the plane portion of the absorber and the viewpoint of further improving a permeation rate of a liquid in the curved portion of the absorber, H10/H1 may be 5.5 or more, 6.0 or more, 6.5 or more, 7.0 or more, 7.5 or more, 8.0 or more, 8.5 or more, 9.0 or more, 9.5 or more, 10.0 or more, 10.5 or more, 11.0 or more, 11.5 or more, 12.0 or more, 12.5 or more, or 13.0 or more. The upper limit of H10/H1 is not particularly limited, and from the viewpoint of further improving a permeation rate of a liquid in the plane portion of the absorber and the viewpoint of further improving a permeation rate of a liquid in the curved portion of the absorber, H10/H1 may be 20.0 or less, 19.5 or less, 19.0 or less, 18.5 or less, 18.0 or less, 17.5 or less, 17.0 or less, 16.5 or less, 16.0 or less, 15.5 or less, 15.0 or less, 14.5 or less, or 14.0 or less. H10/H1 may be 5.0 or more and 20.0 or less, and from the viewpoint of further improving a permeation rate of a liquid in the plane portion of the absorber and the viewpoint of further improving a permeation rate of a liquid in the curved portion of the absorber, H10/H1 may be 6.0 or more and 20.0 or less, 7.0 or more and 19.5 or less, 8.0 or more and 19.0 or less, 8.5 or more and 18.5 or less, 9.0 or more and 18.0 or less, 9.5 or more and 17.5 or less, 10.0 or more and 17.0 or less, 10.5 or more and 16.5 or less, 11.0 or more and 16.0 or less, 11.5 or more and 15.5 or less, 12.0 or more and 15.0 or less, 12.5 or more and 14.5 or less, or 13.0 or more and 14.0 or less.

From the viewpoint of further improving a permeation rate of a liquid in the plane portion of the absorber and the viewpoint of further improving a permeation rate of a liquid in the curved portion of the absorber, H5/H1 may be 4.0 or more, 4.5 or more, 5.0 or more, 5.5 or more, 6.0 or more, 6.5 or more, 7.0 or more, 7.5 or more, 8.0 or more, 8.5 or more, 9.0 or more, 9.5 or more, 10.0 or more, 10.5 or more, 11.0 or more, or 11.5 or more. The upper limit of HS/H1 is not particularly limited, and from the viewpoint of further improving a permeation rate of a liquid in the plane portion of the absorber and the viewpoint of further improving a permeation rate of a liquid in the curved portion of the absorber, H5/H1 may be 20.0 or less, 19.5 or less, 19.0 or less, 18.5 or less, 18.0 or less, 17.5 or less, 17.0 or less, 16.5 or less, 16.0 or less, 15.5 or less, 15.0 or less, 14.5 or less, 14.0 or less, 13.5 or less, 13.0 or less, 12.5 or less, or 12.0 or less. From the viewpoint of further improving a permeation rate of a liquid in the plane portion of the absorber and the viewpoint of further improving a permeation rate of a liquid in the curved portion of the absorber, H5/H1 may be 4.0 or more 20.0 or less, 4.0 or more and 19.5 or less, 4.5 or more and 19.0 or less, 5.0 or more and 18.5 or less, 5.5 or more and 18.0 or less, 6.0 or more and 17.5 or less, 6.5 or more and 17.0 or less, 7.0 or more and 16.5 or less, 7.5 or more and 16.0 or less, 8.0 or more and 15.5 or less, 8.5 or more and 15.0 or less, 9.0 or more and 14.5 or less, 9.5 or more and 14.0 or less, 10.0 or more and 13.5 or less, 10.5 or more and 13.0 or less, 11.0 or more and 12.5 or less, or 11.5 or more and 12.0 or less.

Hn can be adjusted within the above-mentioned range according to, for example, the type of materials used for producing the water-absorbent resin particle, the use amount thereof, and the like. For example, examples of a method for obtaining a water-absorbent resin particle in which Hn and a ratio of each Hn are within the above-mentioned range include a method for providing a coating layer on at least a part of a surface of the water-absorbent resin particle using a coating material. Hn and the ratio of each Hn can be adjusted within the above-mentioned range by adjusting a solubility of the coating layer in water, adjusting the amount of the coating layer in the water-absorbent resin (the use amount of the coating material), using a coating material with low film formation properties, any combination of these methods, or the like.

For example, examples of the method for adjusting H1 and H10/H1 include a method for providing a poorly water-soluble coating layer having an appropriate defect portion where a crosslinked polymer having a water absorption property is exposed. According to the method, in the initial stage of water absorption, rapid water absorption is suppressed by the poorly water-soluble coating layer. Thus, H1 tends to be low. In addition, in a case where the poorly water-soluble coating layer has the appropriate defect portion where the crosslinked polymer having a water absorption property is exposed, water absorption is gradually initiated via the defect portion, and as the water-absorbent resin particle swells, and the crack of the coating layer and the exposed portion of the water-absorbent resin particle are expanded, resulting in a rapid increase in the water absorption capacity to increase H10. Thus, H10/H1 tends to be high. The defect portion where the crosslinked polymer having a water absorption property is exposed may be previously provided, or the coating layer can also be provided with a more water-soluble portion that can be dissolved in the later stage of water absorption to form a defect portion, thereby forming the defect portion after the initiation of water absorption.

A 2-minute value of non-pressurization DW of the water-absorbent resin particle may be 0.1 g/g or more, 0.2 g/g or more, 0.3 g/g or more, 0.4 g/g or more, 0.5 g/g or more, 0.6 g/g or more, 0.7 g/g or more, 0.8 g/g or more, 0.9 g/g or more, or 1.0 g/g or more. The 2-minute value of non-pressurization DW of the water-absorbent resin particle may be 15.0 g/g or less, 14.0 g/g or less, 13.0 g/g or less, 12.0 g/g or less, 11.0 g/g or less, 10.0 g/g or less, 9.0 g/g or less, 8.0 g/g or less, 7.0 g/g or less, 6.0 g/g or less, 5.0 g/g or less, 4.0 g/g or less, 3.0 g/g or less, 2.0 g/g or less, or 1.5 g/g or less. The 2-minute value of non-pressurization DW of the water-absorbent resin particle may be 0.1 g/g or more and 15.0 g/g or less, 0.1 g/g or more and 14.0 g/g or less, 0.2 g/g or more and 13.0 g/g or less, 0.2 g/g or more and 12.0 g/g or less, 0.3 g/g or more and 11.0 g/g or less, 0.3 g/g or more and 10.0 g/g or less, 0.4 g/g or more and 9.0 g/g or less, 0.4 g/g or more and 8.0 g/g or less, 0.5 g/g or more and 7.0 g/g or less, 0.5 g/g or more and 6.0 g/g or less, 0.6 g/g or more and 5.0 g/g or less, 0.7 g/g or more and 4.0 g/g or less, 0.8 g/g or more and 3.0 g/g or less, 0.9 g/g or more and 2.0 g/g or less, or 1.0 g/g or more and 1.5 g/g or less. The non-pressurization DW is a water absorption rate represented by the amount of physiological saline (saline having a concentration of 0.9% by mass) absorbed by the water-absorbent resin particles under no pressurization until a lapse of a predetermined time after the water-absorbent resin particles are brought into contact with the physiological saline. The non-pressurization DW is represented by the absorption amount [g] per 1 g of the water-absorbent resin particles before absorbing the physiological saline. The 2-minute value of non-pressurization DW means the absorption amount 2 minutes after the water-absorbent resin particle is brought into contact with the physiological saline. In a case where the 2-minute value of non-pressurization DW of the water-absorbent resin particle is within the above-mentioned range, the permeation rate of the liquid in the plane portion of the absorber tends to be further improved. A specific method for measuring the non-pressurization DW will be described in detail in Examples.

A shape of the water-absorbent resin particle is not particularly limited, and may be, for example, a substantially spherical shape, a crushed shape, or a granular shape, and a particle formed of primary particles with these shapes in aggregate.

The water-absorbent resin particle may contain a polymer particle. The polymer particle may be a crosslinked polymer formed by polymerization of monomers including an ethylenically unsaturated monomer. The polymer particle may contain an ethylenically unsaturated monomer as a monomer unit. The polymer particle can be produced by a method including a step of polymerizing monomers including ethylenically unsaturated monomers, for example. Examples of methods of polymerization include a reverse phase suspension polymerization method, an aqueous solution polymerization method, a bulk polymerization method, and a precipitation polymerization method.

The ethylenically unsaturated monomer may be a water-soluble ethylenically unsaturated monomer (an ethylenically unsaturated monomer having a solubility of 1.0 g or more in 100 g of water at 25°C). Examples of the water-soluble ethylenically unsaturated monomer include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or two or more kinds thereof may be used in combination.

In a case where the ethylenically unsaturated monomer has an acid group, the ethylenically unsaturated monomer may be used in the polymerization reaction after neutralizing the acid group with an alkaline neutralizing agent. The degree of neutralization of the ethylenically unsaturated monomer by the alkaline neutralizing agent may be 10% to 100% by mol, 50% to 90% by mol, or 60% to 80% by mol of the acid group in the ethylenically unsaturated monomer, for example.

From the viewpoint of industrial availability, the ethylenically unsaturated monomer may include at least one compound selected from the group consisting of (meth)acrylic acid and a salt thereof, acrylamide, methacrylamide, and N,N-dimethyl acrylamide. The ethylenically unsaturated monomer may include at least one compound selected from the group consisting of (meth)acrylic acid and a salt thereof, and acrylamide.

As a monomer for obtaining the water-absorbent resin particle, a monomer other than the above-mentioned ethylenically unsaturated monomer may be used. Such a monomer can be mixed and used with an aqueous solution containing the above-mentioned ethylenically unsaturated monomer, for example. The use amount of the ethylenically unsaturated monomer may be 70% to 100% by mol with respect to the total amount of monomers. The ratio of (meth)acrylic acid and a salt thereof may be 70% to 100% by mol with respect to the total amount of the monomers.

Crosslinking by self-crosslinking occurs during polymerization, but the crosslinking may be induced by using an internal crosslinking agent. When the internal crosslinking agent is used, water-absorbent characteristics (water retention capacity and the like) of the water-absorbent resin particle are easily controlled. The internal crosslinking agent is usually added to a reaction solution during the polymerization reaction.

A polymer on at least a surface layer portion of the polymer particle may be crosslinked by a reaction with a surface crosslinking agent. The surface crosslinking agent may be a compound containing two or more functional groups (reactive functional groups) having reactivity with a functional group derived from the ethylenically unsaturated monomer, for example.

The polymer particle may contain a certain amount of water, and may further contain various additional components therein, in addition to a polymer composed of the ethylenically unsaturated monomers. Examples of the additional components include a gel stabilizing agent and a metal chelating agent.

The water-absorbent resin particle may further contain an inorganic particle adhered to a surface of the polymer particle. Examples of the inorganic particle include a silica particle such as amorphous silica. The amount of the inorganic particles adhered to the surface of the polymer particle may be 0.05% by mass or more, 0.1% by mass or more, 0.15% by mass or more, or 0.2% by mass or more, and may be 5.0% by mass or less, 3.0% by mass or less, 1.0% by mass or less, 0.5% by mass or less, or 0.3% by mass or less, based on the mass of polymer particles. The average particle diameter of the inorganic particles may be 0.1 to 50 $\mu$m, 0.5 to 30 $\mu$m, or 1 to 20 $\mu$m. The average particle diameter can be measured by a pore electric resistance method or a laser diffraction/scattering method depending on the characteristics of particles.

The particle size distribution of the water-absorbent resin particles may be adjusted by performing an operation such as particle size adjustment using classification with a sieve, as necessary. For example, a fraction that passed through a sieve having an opening of 850 $\mu$m but did not pass through a sieve having an opening of 250 $\mu$m may be used as a water-absorbent resin particle.

The water-absorbent resin particle may be a coated resin particle including a polymer particle and a coating layer with which at least a part of a surface of the polymer particle is coated. The coating layer can be formed by a method including a step of coating at least a part of a polymer particle with a coating material to form a coating layer on at least a part of a surface of the polymer particle.

A component constituting the coating layer may be at least one selected from the group consisting of a polymer containing a substituted or unsubstituted alkene as a constituent unit (monomer unit) and fatty acid.

The polymer containing an unsubstituted alkene as a constituent unit may be a homopolymer containing a substituted or unsubstituted alkene as a constituent unit, for example. Examples of the homopolymer containing a substituted or unsubstituted alkene as a constituent unit include polyvinyl alcohol, polystyrene, and the like.

The polymer containing the unsubstituted alkene as a constituent unit may be a copolymer containing only two or more unsubstituted alkenes as constituent units, or may be a copolymer containing one or two or more unsubstituted alkenes and a monomer component other than an unsubstituted alkene as constituent units. The polymer containing an unsubstituted alkene as a constituent unit may be a copolymer containing one unsubstituted alkene and a monomer component other than the unsubstituted alkene as constituent units.

Examples of the unsubstituted alkene include ethylene, propylene, and butene. The unsubstituted alkene may be ethylene and/or propylene, or may be ethylene.

As the monomer component other than the unsubstituted alkene, a water-soluble ethylenically unsaturated monomer may be used. The compounds listed above can be used as the water-soluble ethylenically unsaturated monomer. The water-soluble ethylenically unsaturated monomer may be (meth)acrylic acid and/or a salt thereof.

The copolymer containing the alkene as a constituent unit may be a copolymer containing ethylene and a water-soluble ethylenically unsaturated monomer as constituent units, may be a copolymer containing ethylene and (meth)acrylic acid and/or a salt thereof as a constituent unit, may be a copolymer containing ethylene and an acrylic acid salt as constituent units, or may be a copolymer containing ethylene and sodium acrylate as constituent units (ethylene-sodium acrylate copolymer).

Examples of the fatty acid include at least on selected from the group consisting of vegetable oils and fats such as palm oil, castor oil, rapeseed oil, olive oil, avocado oil, sesame oil and processed products thereof (for example, hardened oils); animal oils and fats such as beef tallow, milk fat, lard, horse fat, egg yolk oil, and processed products thereof; and higher fatty acids such as 12-hydroxystearic acid, palmitoleic acid, oleic acid, and linoleic acid. The fatty acid may be vegetable oils and fats and/or animal oils and fats, or may be vegetable oils and fats, or may be at least one of vegetable oils and fats selected from the group consisting of palm oil, castor oil, rapeseed oil, olive oil, avocado oil, and sesame oil.

The coating layer may contain a polymer containing alkylene oxide as a constituent unit. The coating layer may contain a polymer containing an alkylene oxide as a constituent unit in addition to the polymer containing the above-mentioned substituted or unsubstituted alkene as a constituent unit. In a case where the coating layer contains a polymer (substance A) containing a substituted or unsubstituted alkene as a constituent unit and a polymer (substance B) containing an alkylene oxide as a constituent unit, a ratio (A/B) of a content (% by mass) of the substance A to a content (% by mass) of the substance B may be, for example, 1 to 20, 5 to 15, or 8 to 12. In a case where the coating layer contains a polymer containing a substituted or unsubstituted alkene as a constituent unit and a polymer containing an alkylene oxide as a constituent unit, the polymer containing the alkylene oxide as a constituent unit may be dissolved after the water-absorbent resin particles initiate water absorption and function as a defect portion where a crosslinked polymer having a water absorption property is exposed.

The polymer containing the alkylene oxide as a constituent unit may be a polyalkylene oxide (homopolymer) containing only one alkylene oxide as a constituent unit, may be a polyalkylene oxide (copolymer) containing two or more alkylene oxides as constituent units, or may be a copolymer containing one or two or more alkylene oxides and a monomer component other than an alkylene oxide as constituent units. The polymer containing the alkylene oxide as a constituent unit may be a polyalkylene oxide containing only one alkylene oxide as a constituent unit.

Examples of the alkylene oxide include ethylene oxide or propylene oxide. The alkylene oxide may be ethylene oxide.

Sum up these, the polymer containing the alkylene oxide as a constituent unit may be at least one selected from the group consisting of a homopolymer (polyethylene glycol) containing ethylene oxide as a constituent unit and an ethylene-propylene copolymer containing ethylene oxide and propylene oxide as constituent units, or may be polyethylene glycol.

The coating layer may be a poorly water-soluble coating layer. The poorly water-soluble coating layer means a layer having a dissolved content of 15% by mass or less in 100 g of water at 25°C. A dissolved content of the coating layer in 100 g of water at 25°C may be, for example, 12% by mass or less or 10% by mass or less. The lower limit of a dissolved content of the coating layer in 100 g of water at 25°C is not particularly limited, and may be more than 0% by mass, 1% by mass or more, 5% by mass or more, or 8% by mass or more, for example. The dissolved content of the coating layer in 100 g of water at 25°C is specifically measured by a method described in Examples described later. When the dissolved content of the coating layer is low and/or the amount of the coating layer is large, rapid water absorption by the water-absorbent resin particles in the initial stage of water absorption is easily suppressed.

A ratio of the coating layer in the water-absorbent resin particle may be, for example, 1 to 40 parts by mass with respect to 100 parts by mass of the polymer particle. When the amount of the coating layer is large, rapid water absorption by the water-absorbent resin particles in the initial stage of water absorption is easily suppressed.

The coated resin particle can be obtained by a method including coating at least a part of a polymer particle with a coating material to form a coating layer on at least a part of a surface of the polymer particle. The coating material may contain a liquid medium and the like in addition to the above-mentioned constituent components of the coating layer. Examples of the liquid medium include water and ethers such as tetrahydrofuran. The formation of the coating layer can be performed using a granulator, for example. Examples of the granulator include a fluid bed granulator.

When the fluid bed granulator is used to produce the coated resin particles, first, the polymer particles are injected into a container that is equipped in the fluid bed granulator and can blow hot air from the lower part to previously fluidize the polymer particles. Thereafter, the coating material is sprayed on the fluidized polymer particles while drying. The polymer particles after spraying the coating material may be dried as necessary.

The water retention capacity of physiological saline of the water-absorbent resin particles may be 25 g/g or more, 30 g/g or more, 35 g/g or more, 40 g/g or more, or 45 g/g or more, and may be 60 g/g or less, 55 g/g or less, 50 g/g or less, or 45 g/g or less. The water retention capacity of the water-absorbent resin particles for the physiological saline can be measured by a method described in Examples described later. In a case where the coated resin particles are used as the water-absorbent resin particles, the water retention capacity of the water-absorbent resin particles for physiological saline before forming the coating layer may be within the above-mentioned range.

A water-absorbent resin composition according to an embodiment contains the above-mentioned water-absorbent resin particles (resin particles A), and other water-absorbent resin particles (resin particles B) other than the water-absorbent resin particles. In a case where the water-absorbent resin composition according to the present embodiment is used, the permeation rates in the plane portion and the curved portion of the absorbent article are controlled within a suitable range by appropriately changing the type, mixing ratio, and the like of the resin particles A and B.

In the water-absorbent resin composition, the content of the resin particles A may be, for example, 5 parts by mass or more or 15 parts by mass or more with respect to 100 parts by mass in the total content of the resin particles A and the resin particles B, and may be 95 parts by mass or less, 85 parts by mass or less, 60% by mass or less, 40% by mass or less, 30% by mass or less, or 25% by mass or less.

In the resin particles B, a lower limit value of the water retention capacity with respect to physiological saline (0.9% by mass sodium chloride aqueous solution) may be 25 g/g or more, may be 30 g/g or more, or may be 35 g/g or more. In the resin particles B, an upper limit value of the water retention capacity with respect to physiological saline (0.9% by mass sodium chloride aqueous solution) may be 55 g/g or less, may be 50 g/g or less, or may be 45 g/g or less. The water retention capacity can be measured by a method described in Examples described later.

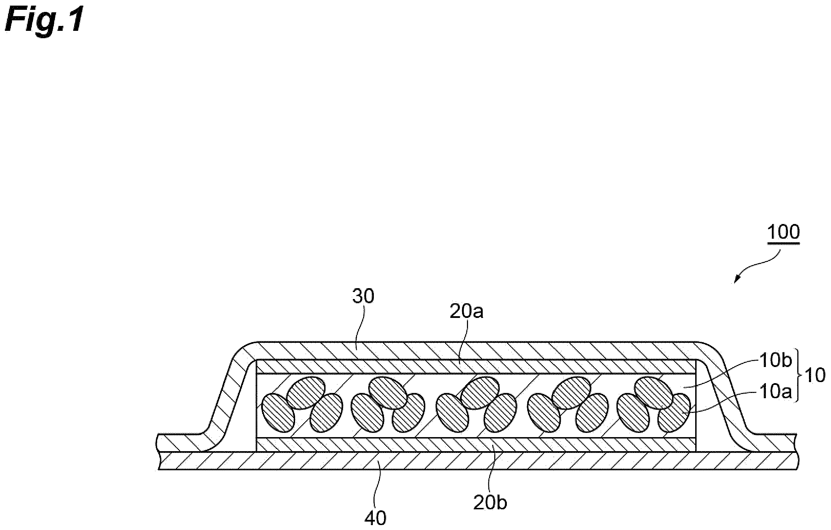

The water-absorbent resin particle is used to form an absorber constituting an absorbent article such as diapers, for example. Fig. 1 is a cross-sectional view showing an example of an absorbent article. An absorbent article 100 shown in Fig. 1 includes a sheet shaped absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that a gap is present between members, but the members may be in close contact with each other without the gap.

The absorber 10 has water-absorbent resin particles 10a according to the present embodiment mentioned above and a fiber layer 10b containing a fibrous material. The water-absorbent resin particles 10a are dispersed in the fiber layer 10b. The water-absorbent resin particles 10a may contain the above-mentioned coated resin particle and other water-absorbent resin particles, for example. In this case, the content of the coated resin particles may be 5 parts by mass or more or 15 parts by mass or more, and may be 95 parts by mass or less or 85 parts by mass or less with respect to 100 parts by mass in the total content of the coated resin particles and the other water-absorbent resin particles, for example.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (on a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, and the like. The core wrap 20a and the core wrap 20b each have, for example, a main surface having the same size as that of the absorber 10.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on the lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 each have, for example, a main surface wider than the main surface of the absorber 10, and outer edges of the liquid permeable sheet 30 and the liquid impermeable sheet 40 each extend around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

It can also be said that one aspect of the present embodiment is a method for improving a permeation rate of an absorbent article that contains water-absorbent resin particles, the method including adjusting H1 and H10/H1 when a lock-up height at an n-minute value is denoted by $H_n$ [cm]. According to the method, the permeation rates of the plane portion and the curved portion of the absorbent article can be improved. The above-mentioned aspects can be applied as a specific aspect of the method.

**EXAMPLES**

Hereinafter, the present invention will be more specifically described with reference to examples. However, the present invention is not limited to these examples.

<Production of Water-absorbent Resin Particle>

[Comparative Example 1]

A round-bottomed cylindrical separable flask with an inner diameter of 11 cm and a volume of 2 L equipped with a reflux cooling device, a dropping funnel, a nitrogen gas introduction tube, and a stirrer (a stirrer blade having two stages of four inclined paddle blades with a blade diameter of 5 cm) was prepared. 293 g of n-heptane (hydrocarbon dispersion medium) and 0.736 g of a maleic anhydride-modified ethylene/propylene copolymer (polymeric dispersant, manufactured by Mitsui Chemicals, Inc., Hi-Wax 1105A) were added into this separable flask to obtain a mixture. The dispersant was dissolved by heating to 80°C while stirring this mixture at a rotation speed of 300 rpm. Thereafter, the mixture was cooled to 55°C.

Subsequently, 92.0 g of an 80.5% by mass acrylic acid aqueous solution (acrylic acid: 1.03 mol) was put into a triangular flask having a volume of 500 mL. Subsequently, while cooling from the outside, 102.2 g of a 30% by mass sodium hydroxide aqueous solution was added dropwise to neutralize 75% by mol of acrylic acid. Thereafter, 0.092 g of hydroxyethyl cellulose (thickener, manufactured by SUMITOMO SEIKA CHEMICALS CO., LTD., HEC AW-15F), 0.0736 g (0.272 mmol) of potassium persulfate (water-soluble radical polymerization initiator), and 0.0101 g (0.0580 mmol) of ethylene glycol diglycidyl ether (internal crosslinking agent), and 32.85 g of ion-exchanged water were added, and then dissolved to prepare a first stage monomer aqueous solution.

The above-mentioned first stage monomer aqueous solution was added to the above-mentioned separable flask, and stirring was then carried out for 10 minutes. 0.736 g of sucrose stearate (surfactant, Mitsubishi-Chemical Foods Corporation, RYOTO Sugar Ester S-370, HLB value: 3) was heat-dissolved in 6.62 g of n-heptane by heating to obtain a surfactant solution. 7.356 g of the obtained surfactant solution was added to the separable flask to obtain a reaction solution. Then, the inside of the separable flask system was sufficiently replaced with nitrogen while stirring the reaction solution at the rotation speed of 550 rpm. Thereafter, the above-mentioned separable flask was immersed in a water bath at 70°C to raise the temperature of the reaction solution, and a first stage polymerization was performed for 10 minutes to obtain a first stage reaction mixture.

Subsequently, 128.8 g of an 80.5% by mass acrylic acid aqueous solution (acrylic acid: 1.44 mol) was put into another triangular flask having a volume of 500 mL. Subsequently, while cooling from the outside, 143.1 g of a 30% by mass sodium hydroxide aqueous solution was added dropwise to neutralize 75% by mol of acrylic acid. Thereafter, 0.1030 g (0.3810 mmol) of potassium persulfate, 0.0116 g (0.0666 mmol) of ethylene glycol diglycidyl ether (internal crosslinking agent), and 0.63 g of ion-exchanged water were added and then dissolved to prepare a second stage monomer aqueous solution.

The first stage reaction mixture was cooled to 25°C while stirring the first stage reaction mixture at a rotation speed of 1000 rpm, and the total amount of the second stage monomer aqueous solution was then added to the first stage reaction mixture to obtain a reaction solution. The inside of the system was sufficiently replaced with nitrogen while stirring the reaction solution. Thereafter, the separable flask was immersed in a water bath at 70°C to raise the temperature of the reaction solution, and the second stage polymerization was performed for 5 minutes to obtain a second stage reaction mixture (polymer particles before surface crosslinking).

After the second stage polymerization, the temperature of the second stage reaction mixture was raised in an oil bath at 125°C, and 254 g of water was extracted to the outside of the system while refluxing n-heptane by azeotropic distillation of n-heptane and water. Subsequently, 0.0884 g (0.5075 mmol) of ethylene glycol diglycidyl ether was added as a surface crosslinking agent, and the mixture was then maintained at 83°C for 2 hours to obtain a dispersion liquid of the polymer particles after surface crosslinking.

Thereafter, the temperature of the dispersion liquid of the polymer particles after the above-mentioned surface crosslinking was raised in an oil bath at 125°C, and n-heptane was evaporated and dried to obtain a dried product. This dried product was passed through a sieve having an opening of 850 $\mu$m to obtain 233.4 g of water-absorbent resin particles (1) in a state in which spherical particles aggregated and were not coated.

Thereafter, 200 g of the above-mentioned water-absorbent resin particles (1) and 0.4 g of TOKUSIL (Oriental Silicas Corporation, product number: NP-S), which is hydrophilic silica, were put into a SUS bottle, and mixed for 30 minutes by a cross rotary mixer to obtain water-absorbent resin particles (2).

[Example 1]

The operations of Comparative Example 1 before the addition of TOKUSIL were repeated, the collected water-absorbent resin particles (1) were classified with a comb having an opening of 250 $\mu$m, and 500 g or more of the water-absorbent resin particles having a particle diameter of 250 to 850 $\mu$m was obtained.

150.0 g of a 25% aqueous dispersion emulsion of an ethylene-sodium acrylate copolymer (SUMITOMO SEIKA CHEMICALS CO., LTD., ZAIKTHENE N) and 3.75 g of a polyethylene glycol (Tokyo Chemical Industry Co., Ltd., PEG 6,000), which serve as a coating material, were diluted with 221.25 g of ion-exchanged water in a polybeaker having an internal volume of 1 L, to prepare a coating material A.

500.0 g of the water-absorbent resin particles was injected to a container of the fluid bed granulator, and hot air at 50°C was blown from the lower part of the container. Subsequently, while drying 375 g of the coating material, the coating material was sprayed on the water-absorbent resin particles whirled up by blowing. After the coating material was sprayed, the water-absorbent resin particles were dried at 50°C for 30 minutes. After the water-absorbent resin particles were dried, 503.2 g of coated resin particles was obtained.

50.0 g of the obtained coated resin particles was spread on a metal tray having a length of 26 cm and a width of 20 cm, and a lid made of an aluminum foil was put thereon. The aluminum foil was perforated, and the coated resin particles were heated for 60 minutes by a hot-air dryer (ADVANTEC, FV-320) set at 80°C to obtain 50.0 g of water-absorbent resin particles of Example 1, each of which has a coating layer.

[Example 2]

100.0 g of a 25% aqueous dispersion emulsion of an ethylene-sodium acrylate copolymer (SUMITOMO SEIKA CHEMICALS CO., LTD., ZAIKTHENE N) and 2.5 g of a polyethylene glycol (Tokyo Chemical Industry Co., Ltd., PEG 6,000) were diluted with 147.5 g of ion-exchanged water, to prepare a coating material B. 50.0 g of water-absorbent resin particles of Example 2, each of which has a coating layer, was obtained in the same manner as in Example 1, except that the coating material B was used instead of the coating material A.

[Example 3]

150 g of polyvinyl alcohol (Kuraray Poval 3-98), 1995.0 g of ion-exchanged water, and 855.0 g of ethanol were mixed to prepare a coating material C. 50.0 g of water-absorbent resin particles of Example 3, each of which has a coating layer, was obtained in the same manner as in Example 1, except that the coating material C was used instead of the coating material A, the blowing temperature of the fluid bed granulator was set to 80°C, and furthermore, the heating temperature of the hot-air dryer was set to 140°C.

[Example 4]

25.0 g of polystyrene (FUJIFILM Wako Pure Chemical Corporation) and 475.0 g of tetrahydrofuran as a solvent were mixed to prepare a coating material D. 506.6 g of water-absorbent resin particles of Example 4, each of which has a coating layer, was obtained in the same manner as in Example 1, except that the coating material D was used instead of the coating material A, the blowing temperature of the fluid bed granulator was set to 40°C, and furthermore, heating by the hot-air dryer was not carried out.

[Example 5]

25.0 g of palm oil (Yamakei Sangyo Co., Ltd.) and 475.0 g of tetrahydrofuran as a solvent were mixed to prepare a coating material E. 505.5 g of water-absorbent resin particles of Example 4, each of which has a coating layer, was obtained in the same manner as in Example 1, except that the coating material E was used instead of the coating material A, the blowing temperature of the fluid bed granulator was set to 40°C, and furthermore, heating by the hot-air dryer was not carried out.

[Comparative Example 2]

A round-bottomed cylindrical separable flask with an inner diameter of 110 mm and an internal volume of 2 L equipped with a reflux cooling device, a dropping funnel, a nitrogen gas introduction tube, and a stirrer (a stirrer blade having two stages of four inclined paddle blades with a blade diameter of 50 mm) was prepared. 40 g of the water-absorbent resin particles (1) produced in Comparative Example 1 and 480 g of n-heptane were mixed in this separable flask.

A polyol aqueous solution obtained by mixing 0.4 g of a polyether polyol (EXCENOL 750ED) with 7.6 g of ion-exchanged water was added to a separable flask, and stirring was then carried out for 30 minutes at room temperature. Thereafter, an isocyanate solution obtained by mixing 0.48 g of tolylene-2,4-diisocyanate with 4.30 g of acetone was added, and stirring was then carried out for 60 minutes at room temperature to cause a sequential polymerization reaction to advance on surfaces of the polymer particles, thereby obtaining a reaction product. Subsequently, the temperature of the reaction product was increased to be evaporated in an oil bath at 125°C to remove n-heptane. Then, the reaction product passed through a sieve having an opening of 850 $\mu$m to obtain 37.3 g of water-absorbent resin particles of Comparative Example 2, which are provided with the polymer particles and the coating layers (polyurethane).

[Comparative Example 3]

25 g of polyvinyl alcohol (Kuraray Poval 3-98), 332.5 g of ion-exchanged water, and 142.5 g of ethanol were mixed to prepare a coating material F. 50.0 g of water-absorbent resin particles of Comparative Example 3, each of which has a coating layer, was obtained in the same manner as in Example 1, except that the coating material F was used instead of the coating material A, the blowing temperature of the fluid bed granulator was set to 80°C, and furthermore, the heating temperature of the hot-air dryer was set to 140°C.

[Comparative Example 4]

233.6 g of water-absorbent resin particles (3) were obtained in the same manner as in Comparative Example 1, except that when the first stage monomer aqueous solution was prepared, the addition amount of ethylene glycol diglycidyl ether (internal crosslinking agent) was set to 0.00828 g (0.0475 mmol), and the azeotropic distillation of n-heptane and water was carried out to extract 260 g of water to the outside of the system while refluxing n-heptane, and the addition amount of TOKUSIL was set to 1.0 g.

[Lock-up Height]

0.200 g of the water-absorbent resin particles of Examples and Comparative Examples, which are obtained by the above-mentioned method, was precisely weighed and arranged in a layered shape on the bottom of an acrylic cylinder having an inner diameter of 2.0 cm and a depth of 8.0 cm, and a height H0 of the particle layer was measured. Thereafter, 20 g of physiological saline was weigh out and poured from the upper part of the acrylic cylinder. The measurement was initiated from the time when the total amount of the physiological saline was poured, a height H1' of the particle layer was read 1 minute after, a height H5' of the particle layer was read 5 minutes after, and a height H10' of the particle layer was read 10 minutes after, and a lock-up height [cm] at an n-minute value was calculated by the following equation.

$$\mathrm{Hn}\ [\mathrm{cm}] = \mathrm{Hn'} - \mathrm{H0}$$

Hn indicates a lock-up height at an n-minute value, Hn indicates a height of the particle layer when n minutes have elapsed, and H0 indicates a height of the particle layer before injecting 20 g of physiological saline.

[Non-pressurization DW]

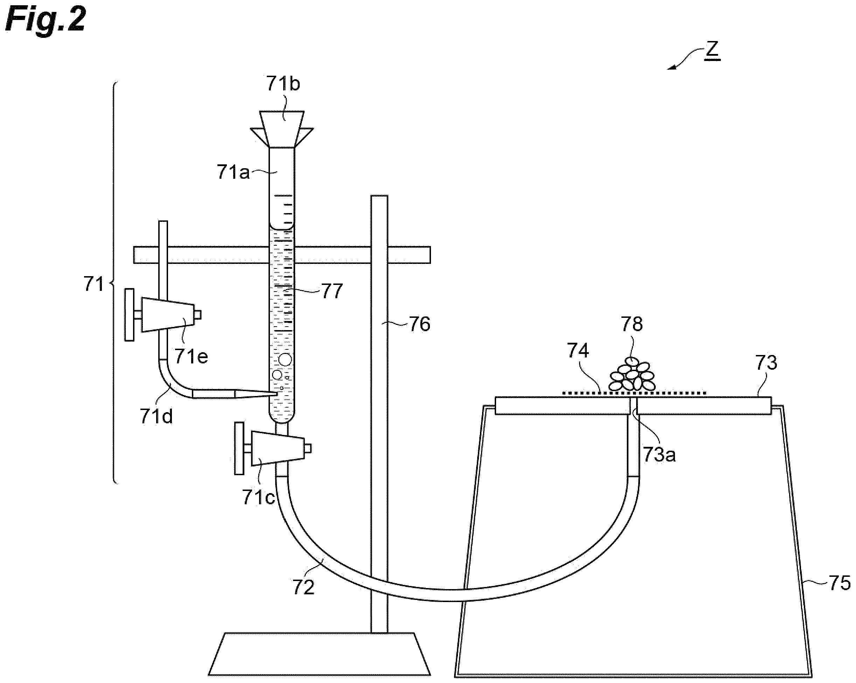

The non-pressurization DW of the water-absorbent resin particles in each of Examples and Comparative Examples obtained by the above-mentioned method was measured by using a measurement device Z shown in Fig. 2.
The measurement device Z has a burette unit 71, a conduit 72, a flat plate-shaped measurement table 73, a nylon mesh 74, a stand 75, and a clamp 76. The burette unit 71 has a burette 71a on which a scale is engraved, a rubber stopper 71b for sealing the opening at the upper part of the burette 71a, a cock 71c connected to the tip end of the lower part of the burette 71a, an air introduction tube 71d connected to the lower part of the burette 71a, and a cock 71e. The burette unit 71 is fixed by the clamp 76. The measurement table 73 has a through hole 73a having the diameter of 2 mm and formed in the central portion thereof, and is supported by the height-variable stand 75. The through hole 73a of the measurement table 73, and the cock 71c of the burette unit 71 are connected by the conduit 72. The inner diameter of the conduit 72 is 6 mm.
The measurement was performed in the environment of the temperature of 25°C and the humidity of 50% ± 10%. First, the cock 71c and the cock 71e of the burette unit 71 were closed, and physiological saline 77 adjusted to 25°C was put into the burette 71a from the opening at the upper part of the burette 71a. The opening of the burette 71a was sealed with the rubber stopper 71b, and the cock 71c and the cock 71e were then opened. The inside of the conduit 72 was filled with the physiological saline 77 to prevent air bubbles from entering. The height of the measurement table 73 was adjusted so that the height of the water surface of the physiological saline 77 reached the inside of the through hole 73a was the same as the height of the upper surface of the measurement table 73. After the adjustment, the height of the water surface of the physiological saline 77 in the burette 71a was read by the scale on the burette 71a, and this position was defined as a zero point (value read at 0 seconds).
The nylon mesh 74 (100 mm × 100 mm, 250 mesh, thickness: about 50 $\mu$m) was laid in the vicinity of the through hole 73a on the measurement table 73, and a cylinder having the inner diameter of 30 mm and the height of 20 mm was placed on the central portion thereof. After uniformly scattering 1.00 g of water-absorbent resin particles 78 in a cylinder, the cylinder was carefully removed to obtain a sample in which the water-absorbent resin particles 78 were dispersed in a circle shape in the central portion of the nylon mesh 74. Then, the nylon mesh 74 on which the water-absorbent resin particles 78 were placed was moved at a high speed to the extent that the water-absorbent resin particles 78 did not dissipate so that the center of the nylon mesh was at the position of the through hole 73a, and the measurement was started. The timing when air bubbles were first introduced from the air introduction tube 71d into the burette tube 71a was defined as the start of water absorption (0 seconds).
The amount of reduction in the physiological saline 77 in the burette 71a (that is, the amount of the physiological saline 77 absorbed by the water-absorbent resin particles 78) was sequentially read by units of 0.1 mL, and the amount of reduction Wc [g] of the physiological saline 77 was read after 2 minutes calculating from the start of water absorption by the water-absorbent resin particles 78. A 2-minute value of non-pressurization DW was obtained from Wc by the following equation. The non-pressurization DW is a water absorption capacity per 1.00 g of the water-absorbent resin particles 78.

$$\text{2-minute value of non-pressurization DW [mL/g]} = \text{Wc/1.00}$$

[Water Retention Capacity]

The measurement was carried out in the environment of 25°C and the humidity of 50%. 2.00 g of the water-absorbent resin particles of Comparative Example 1 or Comparative Example 4 was put into a cotton bag (Cotton broadcloth No. 60, a width of 100 mm × a length of 200 mm), 500 g of 0.9% physiological saline was put into the above-mentioned cotton bag in a beaker having a capacity of 500 ml, the upper part of the cotton bag was bound with a rubber band, and the cotton bag was left to stand for 30 minutes to swell the water-absorbent resin particles. Thereafter, the cotton bag was dehydrated for 1 minute using a dehydrator set at a centrifugal force of 167 G (manufactured by KOKUSAN Co., Ltd., product number: H-122). Thereafter, a mass Wa (g) of the dehydrated cotton bag containing the swollen gel was measured. The same operation was performed without addition of the water-absorbent resin particles, the empty mass Wb (g) at the time when the cotton bag was wet was measured, and the water retention capacity with respect to physiological saline was calculated from the following equation.
Water retention capacity with respect to physiological saline

$$\text{(g/g)} = \text{[Wa - Wb]/amount of water-absorbent resin particles}$$

The water retention capacity of the water-absorbent resin particles of Comparative Example 1 with respect to physiological saline was 42 g/g. The water retention capacity of the water-absorbent resin particles of Comparative Example 4 with respect to physiological saline was 46 g/g.

[Measurement of Dissolved Content]

(Examples 1 and 2)

100 g of a 25% aqueous dispersion emulsion of an ethylene-sodium acrylate copolymer (SUMITOMO SEIKA CHEMICALS CO., LTD., ZAIKTHENE N) and 2.5 g of a polyethylene glycol (Tokyo Chemical Industry Co., Ltd., PEG 6,000) were added to a 200mL polybeaker, to prepare a mixed solution. Thereafter, the mixed solution was put into a Teflon-coating tray having a bottom dimension of 250 × 185 (mm), and covered by aluminum foil as a lid. The aluminum foil was perforated, and heating was carried out with a hot-air dryer (ADVANTEC, FV-320) at 80°C to form a film.
The obtained polymer film was cut with scissors into a 5 mm square to obtain a coating material. The obtained coating material was added to a similar Teflon-coating tray, and covered by aluminum foil as a lid. The aluminum foil was perforated, and heating was carried out with a hot-air dryer (ADVANTEC, FV-320) at 80°C for 2 hours to be dried.
100 g of distilled water put into a 200 mL beaker is adjusted to 25°C and stirred at 600 rpm. The coating material is classified, and 5 g of the coating material remaining on a sieve having an opening of 75 $\mu$m is put into the beaker. The mixture is stirred for 1 hour, and suction-filtration is then carried out by using a 34 $\mu$m stainless steel wire mesh. 60 g of a filtrate obtained by suction filtration is weighed into a preweighed 100 mL beaker and dried with a hot-air dryer (TOYO ROSHI KAISHA, LTD., FV-320) at 140°C for 15 hours, and a mass (Wa) of a solid content of the filtrate is measured. A dissolved content is calculated by the following equation.

$$\text{Amount of precipitation (Wb) in case of 100 g of filtrate (g)} = \text{(Wa/60)} \times 100$$

$$\text{Dissolved content (\% by mass)} = \text{(Wb/5)} \times 100$$

The dissolved content of the coating layer in the water-absorbent resin particle in each of Example 1 and Example 2 was 9.8% by mass.

(Example 3 and Comparative Example 3)

A polyvinyl alcohol (Kuraray Poval 3-98) was classified, and measurement was carried out in the same manner as in Example 1 by using 5 g of the coating material remaining on a sieve having an opening of 75 $\mu$m. The dissolved content of the coating layer in the water-absorbent resin particle in each of Example 3 and Comparative Example 4 was 22% by mass.

(Example 4)

10 g of polystyrene (FUJIFILM Wako Pure Chemical Corporation) was mixed with 190 g of tetrahydrofuran to prepare a solution. Thereafter, the temperature of the hot-air dryer was changed to 40°C to form a film, and measurement was carried out in the same manner as in Example 1 except that the film was dried. The dissolved content of the coating layer in the water-absorbent resin particle of Example 4 was 0% by mass.

(Example 5)

Palm oil (Yamakei Sangyo Co., Ltd.) was classified, and measurement was carried out in the same manner as in Example 1 by using 5 g of the coating material remaining on a sieve having an opening of 75 $\mu$m. The dissolved content of the coating layer in the water-absorbent resin particle of Example 5 was 0% by mass.

(Comparative Example 2)

As a solution for a monomer type A, 36.8 g of a mixed solution (polyol solution) composed of 9.2 g of a polyether polyol (manufactured by AGC Inc., EXCENOL750ED) and 27.6 g of acetone was prepared. In addition, as a solution of a monomer type B, 43.2 g of a mixed solution (isocyanate solution) composed of 10.8 g of tolylene-2,4-diisocyanate and 32.4 g of acetone was prepared.

36.8 g of the solution of the monomer type A and 43.2 g of the solution of the monomer type B were added to a Teflon beaker having an inner diameter of 6.2 cm, and stirring was carried out with a magnetic stirrer until the mixture became uniform to produce a polymer solution. The magnetic stirrer was removed, and the mixture was left to stand at 25°C for 15 hours.

A lid was put by covering with aluminum foil. The aluminum foil was perforated, and a film was formed by heating with a hot-air dryer (ADVANTEC, FV-320) at 40°C for 1 hour, and subsequently heating at 80°C for 1 hour. The obtained polymer film was pulverized by a centrifugal pulverizer (manufactured by Retsch GmbH, ZM200, screen diameter: 1 mm, 6,000 rpm) to obtain a powdery coating material. The obtained coating material was added to a similar Teflon-coating beaker, and covered by aluminum foil as a lid. The measurement was carried out in the same manner as in Example 1, except that aluminum foil was perforated, and heating was carried out with a hot-air dryer (ADVANTEC, FV-320) at 105°C for 2 hours to be dried. The dissolved content of the coating layer in the water-absorbent resin particle of Comparative Example 2 was 0% by mass.

[confirmation of Coating Layer]

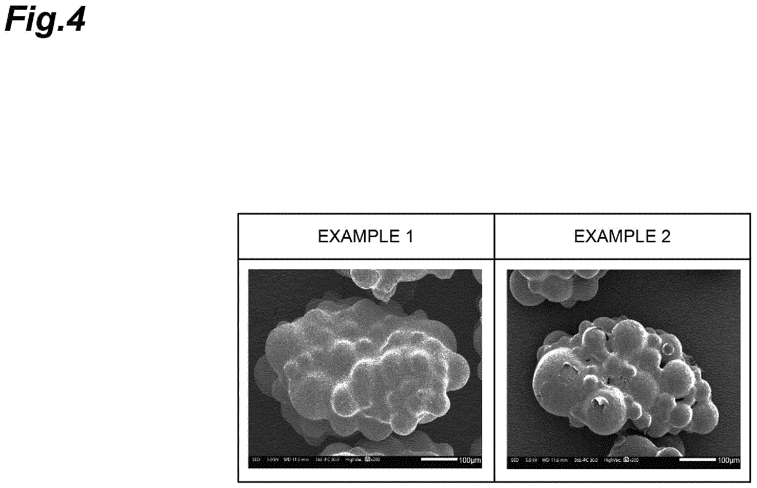

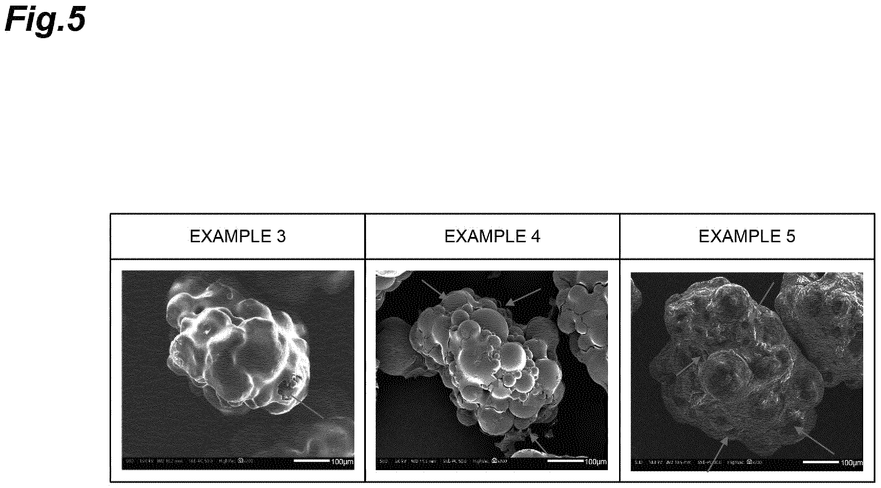

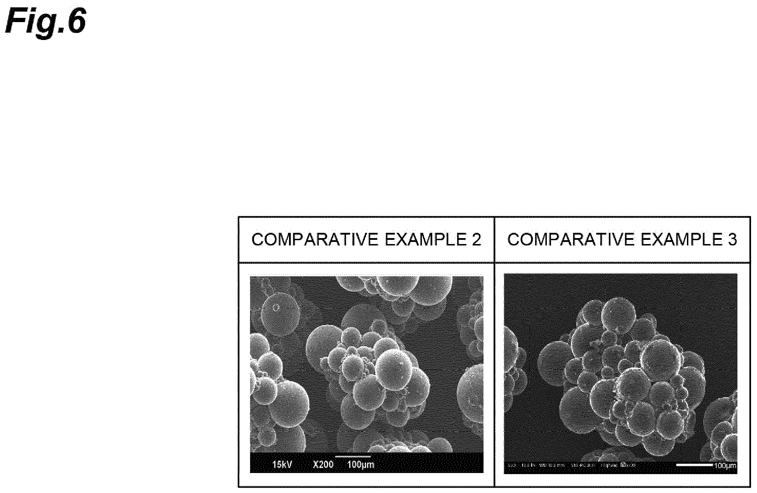

Fig. 4 is scanning electron microscope (SEM) photographs of the water-absorbent resin particles of Examples 1 and 2. Fig. 5 is SEM photographs of the water-absorbent resin particles of Examples 3 to 5. Fig. 6 is SEM photographs of the water-absorbent resin particles of Comparative Examples 2 and 3. As shown in Figs. 4 to 6, it was confirmed that the water-absorbent resin particles of Examples 1 to 5 and Comparative Examples 2 and 3 each had a coating layer. The arrows in the SEM photographs of the water-absorbent resin particles of Examples 3 to 5 indicate the defect portions (portions that can function as a water channel at the later stage of water absorption) of the coating layers in which the crosslinked polymers having a water absorption property are exposed. Although No defect portions were confirmed in the SEM photographs of the water-absorbent resin particles of Examples 1 and 2, it is presumed that when the water-absorbent resin particles come into contact with water, polyethylene glycol (more water-soluble portion) constituting the coating layer is eluted, resulting in subsequent formation of the defect portions.

<Evaluation>

In the flat test and U-shaped test, the permeation rates were evaluated for absorbers produced using the water-absorbent resin particles of Examples 1 to 5 and Comparative Examples 1 to 4.

(Production of Water-Absorbent Resin Particles Used for Absorber)

Mixed particles in a total of 12 g of 9.6 g of the water-absorbent resin particles (2) produced in Comparative Example 1 and 2.4 g of the coated resin particles produced in Examples 1 to 5 and Comparative Examples 2 and 3 were used as absorbers. In addition, 12 g of the water-absorbent resin particles (2) was used alone without mixing in the absorber of Comparative Example 1, and 12 g of the water-absorbent resin particles (3) were used alone in the absorber of Comparative

Example 4.

(Preparation of Absorber)

12 g of the above-mentioned mixed resin particles for the evaluation of an absorber and 8.0 g of crushed pulp (manufactured by Rayonier Inc., trade name: Rayfloc) were uniformly mixed by air papermaking to produce an absorber core having a size of 40 cm × 12 cm. Subsequently, an absorber (particle content: 60% by mass) was produced by applying a load of 141 kPa to the entire absorber core for 30 seconds and pressing the absorber core in a state in which the upper and lower parts of the absorber core sandwiched between two sheets of tissue paper (basis weight: 16 $g/m^2$) having the same size as the absorber core.

(Production of Absorbent Article for Evaluation)

An air through type porous liquid permeable sheet (basis weight: 22 $g/m^2$) made of polyethylene having the same size as the absorber was disposed on the upper surface of the above-mentioned absorber, a liquid impermeable sheet (basis weight: 22 $g/m^2$) made of polyethylene having the same size as the absorber was disposed on the lower surface of the absorber to sandwich the absorber, thereby obtaining an absorbent article for the evaluation.

(Preparation of Test Solution)

100.0 g of sodium chloride, 3.0 g of calcium chloride dihydrate, 6.0 g of magnesium chloride hexahydrate, and a mixture of 1.0 g of Triton X-100 (polyoxyethylene(10) octylphenyl ether, manufactured by FUJIFILM Wako Pure Chemical Corporation) and 99.0 g of ion-exchanged water, and 9866.0 g of ion-exchanged water were put in a container having an internal volume of 10 L, and each component was then completely dissolved. Subsequently, the mixed solution was colored with a small amount of Blue No. 1 to prepare a test solution.

(Measurement of Permeation Rate of Flat Test)

The absorbent article for an evaluation was placed on a horizontal table. A liquid injection cylinder having an opening with an inner diameter of 3 cm was placed on the center of the absorbent article for evaluation, and 80 mL of the above-mentioned test solution was injected into the cylinder at one time to measure the absorption rate. The same operation was performed a total of five times at intervals of 30 minutes, and a total of the five absorption rates was obtained as a permeation rate (flat) [second].

(Measurement of Permeation Rate of U-shaped Test)

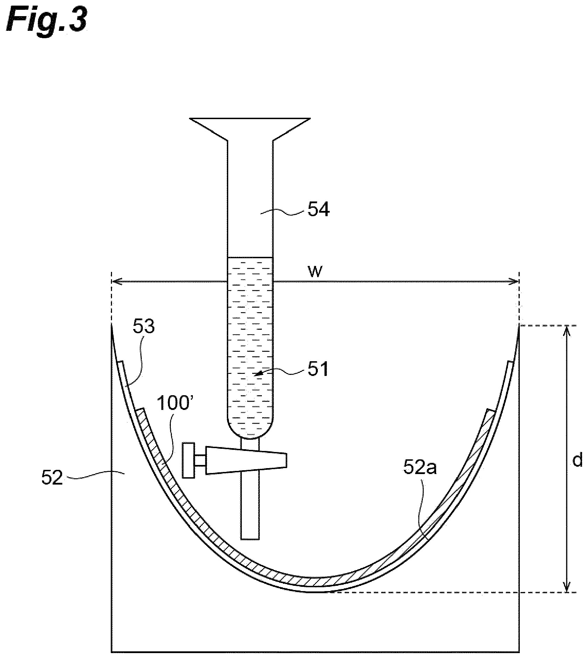

Fig. 3 is a schematic diagram showing a test method for evaluating a permeation rate of a liquid in a curved portion of an absorber. This test was performed in the environment of the temperature of 25°C ± 2°C and the humidity of 50% ± 10%. The U-shaped instrument 52 shown in Fig. 3 is an acrylic resin molded body that has a curved surface 52a with a U-shaped cross section and has an opening on the upper portion. The curved surface 52a has an opening width W of 22 cm, a depth d of 18.5 cm, and a length of 10 cm. A back sheet 53, which was collected from a commercially available children's diaper (DAIO PAPER CORPORATION, trade name: GOO.N Plus sensitive skin design, tape type L size) in Japan, was placed on the curved surface 52a. The absorbent article 100' for evaluation was bonded onto the back sheet 53 with a spray glue so that the center thereof was located at the innermost portion of the curved surface 52a. A liquid injection burette 54 having a volume of 200 mL was fixed 5 cm in front of the center of the absorbent article 100' for evaluation and 1 cm above the absorbent article 100' for evaluation. 80 mL of a test solution 51 was put into the liquid injection burette 54, and injected at a rate of 10 mL/sec. A time from when the test solution 51 first reaches the absorbent article 100' for evaluation to when the test solution 51 completely disappears from the absorbent article 100' for evaluation is defined as a first permeation time (second). The same operation was performed a total of five times at intervals of 30 minutes, and a total of the five absorption rates was obtained as a permeation rate (U-shaped) [second].

[Table 1]

| No. | Water-absorbent resin particle performance | | | | | | Evaluation | |
|---|---|---|---|---|---|---|---|---|
| | H1 | H5 | H10 | H5/H1 | H10/H1 | DW (g/g) 2 min | Permeation rate of flat portion | Permeation rate of U-shaped portion |
| Example 1 | 0.4 | 4.7 | 5.4 | 11.8 | 13.5 | 1.0 | 270 | 685 |
| Example 2 | 0.6 | 4.6 | 5.4 | 7.7 | 9.0 | 1.1 | 280 | 688 |
| Example 3 | 0.2 | 2.6 | 3.5 | 13.0 | 17.5 | 0.1 | 279 | 772 |
| Example 4 | 0.5 | 2.8 | 4.3 | 5.6 | 8.6 | 0.1 | 281 | 731 |
| Example 5 | 0.3 | 2.4 | 4.2 | 8.0 | 14.0 | 0.1 | 284 | 698 |
| Comparative Example 1 | 1.7 | 5 | 5.1 | 2.9 | 3.0 | 17.0 | 306 | 617 |
| Comparative Example 2 | 0.6 | 1.4 | 2.3 | 2.3 | 3.8 | 0.1 | 260 | 1469 |
| Comparative Example 3 | 1.6 | 4.6 | 4.9 | 2.9 | 3.1 | 2.3 | 301 | 630 |
| Comparative Example 4 | 3.2 | 5.8 | 5.8 | 1.8 | 1.8 | 42.0 | 315 | 581 |

As shown in Table 1, it was confirmed that the absorbent article containing the water-absorbent resin particles of each of the examples had a sufficiently high permeation rate in both the plane portion and the curved portion.

According to the findings found by the present inventors of the present invention, the usability of the absorbent article hardly varies when a permeation rate of the flat portion based on the above-mentioned measurement method is 300 seconds or less (preferably 280 seconds or less), and a permeation rate of the U-shaped portion is 800 seconds or less (preferably 700 seconds or less). It was confirmed that each of Examples satisfy this condition.

**Reference Signs List**

10: Absorber,
10a: Water-absorbent resin particle,
10b: Fiber layer,
20a, 20b: Core wrap,
30: Liquid permeable sheet,
40: Liquid impermeable sheet,
51: Test solution,
52: U-shaped instrument,
52a: Curved surface,
53: Back sheet,
54: Burette for liquid injection,
71: Burette unit,
71a: Burette,
71b: Rubber stopper,
71c, 71e: Cock,
71d: Air introduction tube,
72: Conduit,
73: Measurement table,
74: Nylon mesh,
73a: Through hole,
75: Stand,

76: Clamp,
77: Physiological saline,
100: Absorbent article, 100': Absorbent article for evaluation,
Z: Measurement device.

## Claims

1. A water-absorbent resin particle comprising

   H1 of 1.0 cm or less and H10/H1 of 5.0 or more when a lock-up height at an n-minute value is denoted by Hn [cm], wherein the lock-up height at the n-minute value is measured by the following procedure:

   (1) 0.200 g of water-absorbent resin particles is disposed over an entire bottom surface in a cylinder having an inner diameter of 2.0 cm and a depth of 8.0 cm to form a particle layer, and a height of the particle layer is denoted by H0 [cm];
   (2) 20 g of physiological saline is injected to the particle layer to swell the particle layer; and
   (3) a height of the particle layer when n minutes have passed from the injection of a total amount of the physiological saline is recorded as Hn', and a lock-up height Hn [cm] at an n-minute value is calculated from an expression represented by Hn' - H0.

2. The water-absorbent resin particle according to claim 1,
   wherein a 2-minute value of non-pressurization DW is 15.0 or less.

3. The water-absorbent resin particle according to claim 1 or 2,
   wherein the water-absorbent resin particle is a coated resin particle comprising a polymer particle, and a coating layer covering at least a part of a surface of the polymer particle.

4. The water-absorbent resin particle according to claim 3,
   wherein the coating layer is a poorly water-soluble coating layer.

5. A water-absorbent resin composition comprising:

   the water-absorbent resin particle according to any one of claims 1 to 4; and
   a water-absorbent resin particle other than the water-absorbent resin particle.

6. An absorber comprising the water-absorbent resin particle according to any one of claims 1 to 4 or the water-absorbent resin composition according to claim 5.

7. An absorbent article comprising the absorber according to claim 6.

8. The absorbent article according to claim 7,
   wherein the absorbent is a diaper.

*Fig.1*

**Fig.2**

*Fig.3*

## Fig.4

| EXAMPLE 1 | EXAMPLE 2 |
|---|---|

*Fig.5*

| EXAMPLE 3 | EXAMPLE 4 | EXAMPLE 5 |
|---|---|---|
| | | |

# Fig.6

| COMPARATIVE EXAMPLE 2 | COMPARATIVE EXAMPLE 3 |

## INTERNATIONAL SEARCH REPORT

| | International application No. |
|---|---|
| | **PCT/JP2022/017873** |

### A. CLASSIFICATION OF SUBJECT MATTER

***C08J 3/12***(2006.01)i
FI:  C08J3/12 Z CEY

According to International Patent Classification (IPC) or to both national classification and IPC

### B. FIELDS SEARCHED

Minimum documentation searched (classification system followed by classification symbols)

C08J3/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 57-168921 A (SUMITOMO KAGAKU KOGYO KK) 18 October 1982 (1982-10-18) claims, p. 2, upper left column, lines 10-17, p. 3, upper left column, line 12 to lower right column, line 4, example 4 | 1-3, 5-8 |
| X | JP 1-261250 A (TSURUGA CEMENT KK) 18 October 1989 (1989-10-18) claims, p. 2, lower left column, line 9 to p. 4, upper left column, line 9, examples | 1-7 |
| X | JP 2-242858 A (SANYO CHEMICAL IND LTD) 27 September 1990 (1990-09-27) claims, p. 2, lower right column, line 7 to p. 3, upper left column, line 19, p. 3, upper right column, line 15 to lower left column, line 12, examples | 1-8 |
| X | JP 56-159232 A (KURARAY CO) 08 December 1981 (1981-12-08) claims, p. 1, lower left column, line 12 to lower right column, line 12, p. 2, upper right column, line 10 to p. 3, upper right column, line 6, examples | 1-3, 5-8 |
| A | JP 56-115259 A (KURARAY CO) 10 September 1981 (1981-09-10) entire text | 1-8 |
| A | JP 3-285918 A (TOKAI RUBBER IND LTD) 17 December 1991 (1991-12-17) entire text | 1-8 |

✓ Further documents are listed in the continuation of Box C.   ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **24 June 2022** | **05 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2022/017873** |

| C. | DOCUMENTS CONSIDERED TO BE RELEVANT | |
| --- | --- | --- |
| Category* | Citation of document, with indication, where appropriate, of the relevant passages | Relevant to claim No. |
| A | JP 11-347402 A (SANYO CHEMICAL IND LTD) 21 December 1999 (1999-12-21) entire text | 1-8 |

Form PCT/ISA/210 (second sheet) (January 2015)

INTERNATIONAL SEARCH REPORT
Information on patent family members

International application No.

**PCT/JP2022/017873**

| Patent document cited in search report | | | Publication date (day/month/year) | Patent family member(s) | | | Publication date (day/month/year) |
|---|---|---|---|---|---|---|---|
| JP | 57-168921 | A | 18 October 1982 | (Family: none) | | | |
| JP | 1-261250 | A | 18 October 1989 | (Family: none) | | | |
| JP | 2-242858 | A | 27 September 1990 | (Family: none) | | | |
| JP | 56-159232 | A | 08 December 1981 | (Family: none) | | | |
| JP | 56-115259 | A | 10 September 1981 | (Family: none) | | | |
| JP | 3-285918 | A | 17 December 1991 | (Family: none) | | | |
| JP | 11-347402 | A | 21 December 1999 | JP | 11-347403 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014046020 A